# EUROPÄISCHE PATENTSCHRIFT

(11) **EP 1 294 670 B1**
(45) Veröffentlichungstag und Bekanntmachung des Hinweises auf die Patenterteilung: **07.12.2005**
(21) Anmeldenummer: 01967099.1
(22) Anmeldetag: 27.06.2001
(51) Int. Cl.: C07C 45/50, C07C 47/02

(54) **VERFAHREN ZUR HERSTELLUNG VON HYDROFORMYLIERUNGSPRODUKTEN VON OLEFINEN MIT 2 BIS 8 KOHLENSTOFFATOMEN**
METHOD FOR PRODUCING HYDROFORMYLATION PRODUCTS OF OLEFINS WITH 2 TO 8 CARBON ATOMS
PROCEDE DE PRODUCTION DE PRODUITS D'HYDROFORMYLATION D'OLEFINE AYANT ENTRE 2 ET 8 ATOMES DE CARBONE

(30) Priorität: 28.06.2000 DE 10031517
(43) Veröffentlichungstag der Anmeldung: 26.03.2003
(73) Patentinhaber: BASF Aktiengesellschaft, 67056 Ludwigshafen (DE)
(72) Erfinder: RICHTER, Wolfgang, 67157 Wachenheim (DE); KROKOSZINSKI, Roland, 67273 Weisenheim a. Berg (DE); MÜLLER, Rolf, 67125 Dannstadt-Schauernheim (DE); GEISSLER, Bernhard, 67281 Kirchheim (DE)
(74) Vertreter: Kinzebach, Werner
(86) Internationale Anmeldenummer: PCT/EP2001/007343
(87) Internationale Veröffentlichungsnummer: WO 2002/002496

(56) Entgegenhaltungen:
- EP-A- 0 254 180
- FR-A- 2 249 061
- US-A- 2 846 464
- US-A- 4 711 968

## Beschreibung

Die vorliegende Erfindung betrifft ein Verfahren zur Herstellung von Hydroformylierungsprodukten von Olefinen mit 2 bis 8 Kohlenstoffatomen.

Die Hydroformylierung oder Oxo-Synthese ist ein wichtiges großtechnisches Verfahren zur Herstellung von Aldehyden aus Olefinen, Kohlenmonoxid und Wasserstoff. Diese Aldehyde können gegebenenfalls im gleichen Arbeitsgang oder nachfolgend in einem getrennten Hydrierschritt mit Wasserstoff zu den entsprechenden Alkoholen hydriert werden. Die Hydroformylierung erfolgt in Anwesenheit von homogen im Reaktionsmedium gelösten Katalysatoren. Als Katalysatoren werden dabei im Allgemeinen Verbindungen oder Komplexe von Metallen der VIII. Nebengruppe, speziell Co-, Rh-, Ir-, Pd-, Pt- oder Ru-Verbindungen bzw. -komplexe eingesetzt, die unmodifiziert oder z. B. mit amin- oder phosphinhaltigen Verbindungen modifiziert sein können.

Der Reaktionsaustrag aus dem Hydroformylierungsreaktor enthält neben dem Hydroformylierungsprodukt in der Regel nicht umgesetztes Olefin. Dieses wird vom Hydroformylierungsprodukt abgetrennt und zusammen mit frischem Kohlenmonoxid und Wasserstoff wieder dem Hydroformylierungsreaktor zugeführt. Mit dem zurückgeführten Olefin werden allerdings auch die mit dem Olefin eingeführten oder durch Nebenreaktionen gebildeten Inertkomponenten, z. B. gesättigte Kohlenwasserstoffe, die der Hydroformylierung nicht zugänglich sind, in den Reaktor zurückgeführt. Um zu verhindern, dass die Konzentration der Inertkomponenten im Hydroformylierungsreaktor kontinuierlich ansteigt und Werte erreicht, bei denen die Hydroformylierungsreaktion zum Erliegen kommt, muss kontinuierlich ein Teilstrom des rückgeführten Olefins aus dem Verfahren ausgeleitet werden, um die Inertkomponenten aus dem System zu entfernen.

Der Ausleitstrom besteht jedoch nur zu einem Teil aus Inertkomponenten. Einen wesentlichen Teil bildet nicht umgesetztes Olefin, das somit für die Umsetzung verloren geht. Um den Ausleitstrom klein und die damit verbundenen Verluste gering zu halten, wird im Allgemeinen ein Olefinzulauf hoher Reinheit eingesetzt.

Es ist zwar technisch möglich, aus dem Ausleitstrom den gesättigten Kohlenwasserstoff abzutrennen und lediglich das nicht umgesetzte Olefin bzw. einen Olefin-angereicherten Strom in die Reaktionszone zurückzuführen. Die Trennung von Olefinen und gesättigten Kohlenwasserstoffen gleicher Kohlenstoffzahl erfordert jedoch aufwendige Vorrichtungen, die mit einem erheblichen Investitionsaufwand verbunden sind.

Die EP-A 0 648 730 offenbart ein Verfahren zur Herstellung eines aus Propylen gewonnenen Oxoprodukts, bei dem aus dem Produktstrom einer Propylen-Hydroformylierung ein Gasstrom abgetrennt wird, der nicht umgesetztes Propylen und Propan enthält. Durch selektive Adsorption von Propylen an ein Adsorptionsmittel und anschließende Desorption wird ein Propylen-angereicherter Gasstrom erhalten, der zumindest teilweise in die Reaktionszone zurückgeführt wird. Die alternierenden Adsorptions- und Desorptionscyclen erfordern periodische Druck- und/oder Temperaturwechsel. Die dazu benötigten Apparaturen sind aufwendig und störanfällig.

Olefine, wie Ethylen, Propylen und Butene, werden in großem Umfang durch thermische und/oder katalytische Crackung und/oder Dehydrierung eines Kohlenwasserstoffeinsatzmaterials, wie Naphtha, Erdgas, Isobutan usw., hergestellt. Aus den rohen Spaltprodukten werden die einzelnen Olefine in der gewünschten Reinheit isoliert.

Die Aufgabe der vorliegenden Erfindung besteht darin, einen Verfahrensverbund zur Herstellung und Hydroformylierung von Olefinen anzugeben, bei dem Investitionskosten-intensive Anlagen zur Trennung von Olefinen und gesättigten Kohlenwasserstoffen möglichst optimal genutzt werden.

Diese Aufgabe wird erfindungsgemäß durch ein Verfahren zur Hydroformylierung von Olefinen mit 2 bis 8 Kohlenstoffatomen gelöst, bei dem man
a) ein Kohlenwasserstoff-Einsatzmaterial in eine Crack/Dehydrierzone einspeist und einer thermischen und/oder katalytischen Crackung und/oder Dehydrierung unter Erhalt eines olefinhaltigen Spaltgases unterwirft,
b) das Spaltgas oder Fraktionen davon einer Auftrennung in wenigstens einen Cᵢ-Olefin-angereicherten Kohlenwasserstoffstrom und wenigstens einen Cᵢ-Olefin-abgereicherten Kohlenwasserstoffstrom unterzieht,
c) den Cᵢ-Olefin-angereicherten Kohlenwasserstoffstrom mit Kohlenmonoxid und Wasserstoff in eine Hydroformylierungszone einspeist und in Gegenwart eines Hydroformylierungskatalysators umsetzt,
d) aus dem Austrag aus der Hydroformylierungszone einen im Wesentlichen aus nicht umgesetztem Cᵢ-Olefin und gesättigtem Cᵢ-Kohlenwasserstoff bestehenden Strom abtrennt,
e) den im Wesentlichen aus nicht umgesetztem Cᵢ-Olefin und gesättigtem Cᵢ-Kohlenwasserstoff bestehenden Strom zumindest teilweise in den Schritt b) zurückführt, wobei i für eine ganze Zahl von 2 bis 8 steht.

In der Regel führt man den Cᵢ-Olefin-abgereicherten Kohlenwasserstoffstrom zumindest teilweise in die Crack/Dehydrierzone zurück. Die thermische Crackung erfolgt vorzugsweise in Gegenwart von Wasserdampf.

Das erfindungsgemäße Verfahren umfasst die Stufen der Herstellung eines olefinhaltigen Spaltgases aus einem Kohlenwasserstoff-Einsatzmaterial (z. B. in einem Steamcracker oder einer Kohlenwasserstoff-Dehydrieranlage), der Auftrennung des Spaltgases unter Gewinnung einer an einem einzelnen Olefin angereicherten Fraktion und der Hydroformylierung dieser Fraktion. Der im Schritt b) anfallende Mengenstrom an Cᵢ-Olefin-angereichertem Kohlenwasserstoff kann auch aufgeteilt werden und nur eine Teilmenge hydroformyliert werden. Ein wesentliches Kennzeichen der Erfindung besteht darin, dass ein vom Hydroformylierungsaustrag abgetrennter Strom, der im Wesentlichen aus nicht umgesetztem Olefin und gesättigtem Kohlenwasserstoff gleicher Kohlenstoffzahl besteht, unmittelbar zurück in die Stufe der Auftrennung des olefinhaltigen Spaltgases geleitet und gemeinsam mit dem Spaltgas oder Fraktionen davon aufgetrennt wird. Für den gemeinsamen Betrieb der Olefinisolierung aus dem Spaltgas und der selektiven Rückführung von nicht umgesetztem Olefin in die Hydroformylierung ist die Errichtung lediglich einer Trennanlage erforderlich. Da Anlagen für die Trennung von Olefinen und gesättigten Kohlenwasserstoffen sehr aufwendig sind, ist damit eine erhebliche Ersparnis von Investitionskosten verbunden. Es wird in der vorliegenden Beschreibung einheitlich der Begriff "Spaltgas" für ein Olefine und gesättigte Kohlenwasserstoffe enthaltenes Reaktionsprodukt verwendet, ohne Rücksicht darauf, ob es durch Crackung oder Dehydrierung erhalten wird.

Geeignete Kohlenwasserstoff-Einsatzmaterialien für das erfindungsgemäße Verfahren sind z. B. Naphtha, Erdgas oder Fraktionen davon, Propan, Isobutan oder sonstige Kohlenwasserstoffströme, die z. B. bei technischen Herstellverfahren als Nebenprodukt- oder Abfallströme anfallen.

Das durch Crackung/Dehydrierung erhaltene Spaltgas enthält im Allgemeinen ein Gemisch von Olefinen und gesättigten Kohlenwasserstoffen, die insbesondere bei Crack-Spaltgas unterschiedliche Kohlenstoffzahlen aufweisen können. Das Spaltgas wird einer Auftrennung in wenigstens einen Cᵢ-Olefin-angereicherten und wenigstens einen Cᵢ-Olefin-abgereicherten Kohlenwasserstoffstrom unterzogen. Sind Kohlenwasserstoffe unterschiedlicher Kohlenstoffzahlen im Spaltgas vorhanden, isoliert man in der Regel aus dem Gemisch zuerst eine Ci-Kohlenwasserstofffraktion, die dann weiter in einen Olefin-angereicherten und einen Olefin-abgereicherten Strom aufgetrennt wird. So wird bei der Auftrennung von Spaltgas, das durch Naphtha-Dampfspaltung erhalten wird, eine C₂- und eine C₃-Kohlenwasserstofffraktion gewonnen, die dann ihrerseits in Ethylen und Ethan bzw. Propylen und Propan weiter aufgetrennt werden. Die Auftrennung umfasst in der Regel wenigstens einen Rektifikationsschritt.

Der Index i steht für eine ganze Zahl von 2 bis 8, vorzugsweise 2 bis 4, insbesondere 3. Mit den erfindungsgemäßen Verfahren können insbesondere die Hydroformylierungsprodukte von Ethylen, Propylen, 1-Buten und/oder 2-Buten hergestellt werden, wobei Propylen besonders bevorzugt ist.

Das erfindungsgemäße Verfahren kann z. B. in einem bestehenden Verfahren zur Hydroformylierung von Propylen, das durch Naphtha-Dampfspaltung in einem Steamcracker erhalten wird, implementiert werden, indem man einen aus dem Austrag aus der Hydroformylierungszone abgetrennten, im Wesentlichen aus nicht umgesetztem Propylen und Propan bestehenden Strom direkt in die C₃-Kolonne des Steamcrackers (siehe unten) leitet.

Andererseits ist es z. B. möglich, in die Hydroformylierungszone einen durch Propan-Dehydrierung gewonnenen Propylen-haltigen Strom, der z. B. noch 0,5 bis 40 Gew.-% Propan enthalten kann, einzuspeisen und den vom Austrag aus der Hydroformylierungszone abgetrennten, aus nicht umgesetztem Propylen und Propan bestehenden Strom wieder in den Aufarbeitungsteil der Propan-Dehydrierungsanlage zu leiten, wobei der dabei gewonnene Propylen-abgereicherte Strom mit Vorteil als Einsatzmaterial zurück in die Dehydrierungszone geleitet wird.

Der Cᵢ-Olefin-angereicherte Kohlenwasserstoffstrom enthält im Allgemeinen wenigstens 60 Gew.-%, vorzugsweise wenigstens 70 Gew.-%, insbesondere wenigstens 90 Gew.-%, z. B. 90 bis 97 Gew.-% Cᵢ-Olefin, wobei der Rest im Wesentlichen aus gesättigtem Cᵢ-Kohlenwasserstoff besteht. Der Cᵢ-Olefin-abgereicherte Kohlenwasserstoffstrom enthält in der Regel weniger als 15 Gew.-%, meist weniger als 5 Gew.-% Cᵢ-Olefin, wobei die Hauptmenge aus gesättigtem Cᵢ-Kohlenwasserstoff besteht.

Die einzelnen Schritte des erfindungsgemäßen Verfahrens sind an sich bekannt und in ihrer spezifischen Ausgestaltung nicht Gegenstand der Erfindung. Sie werden im Folgenden anhand zweckmäßiger oder bevorzugter Ausgestaltungen näher erläutert.

Bei der thermischen Crackung handelt es sich um eine radikalische Spaltung von Kohlenwasserstoffen, die zumeist unter Druck ausgeführt wird und bei etwa 400 bis 500 °C einsetzt. Die thermische Spaltung beginnt mit der Homolyse einer C-C-Bindung unter Ausbildung zweier freier Radikale. Beide Alkylradikale können nun aus einem weiteren Kohlenwasserstoffmolekül ein Wasserstoffatom abstrahieren und damit ein neues freies Radikal und ein kürzerkettiges Alkan erzeugen. Beide Ursprungsradikale oder auch das neu erzeugte Radikal können einer β-Spaltung unter Bildung eines Olefinmoleküls und eines kürzerkettigen Alkylradikals unterliegen. Die Spaltvorgänge umfassen also Veränderungen im H₂-Gehalt und im Kohlenstoffskelett. Die Herstellung von niedermolekularen Olefinen wird begünstigt, wenn die thermische Spaltung bei hoher Temperatur, kurzer Verweilzeit und niedrigem Partialdruck durchgeführt wird. Um den Partialdruck der Kohlenwasserstoffe zu erniedrigen, mischt man ein Fremdgas, zumeist Wasserdampf, dem zu pyrolisierenden Kohlenwasserstoff-Einsatzmaterial bei ("Dampfspaltung" oder "Steam-Cracken").

Lediglich zu Zwecken der Veranschaulichung lässt sich die Arbeitsweise der Naphthaspaltung in folgende Einzelschritte unterteilen:
(1) Spaltung von Naphtha in Röhrenöfen,
(2) Quenchung, d. h. Abschreckung des Spaltgases,
(3) Verdichtung und Reinigung des Spaltgases und
(4) Trocknung, Kühlung und Tieftemperatur-Destillation.

Naphtha wird nach einer Vorverdampfung mit überhitztem Wasserdampf in die z. B. 50 bis 200 m langen und 80 bis 120 mm weiten Röhren des Spaltofens eingebracht. Moderne High Severity-Cracköfen umfassen in der Regel senkrecht angeordnete Chrom-Nickelrohre. Sie werden direkt durch die Verbrennung von Heizgasen oder -ölen auf etwa 1050 °C an der heißesten Stelle beheizt. Beim sogenannten ACR-Verfahren werden in einem keramisch ausgekleideten Reaktor Temperaturen von bis zu 2000 °C und Drücke von 3,5 bar erreicht.

Die Spaltprodukte treten mit hoher Temperatur aus der Spaltstrecke aus und müssten zur Vermeidung von Folgereaktionen schnell auf etwa 300 °C abgeschreckt (gequencht) werden. Dies geschieht zunächst indirekt unter Dampferzeugung in Quenchkühlern und anschließend durch Einspritzen von Quenchöl. Anschließend werden Prozesswasser und Pyrolysebenzin abgetrennt und die gasförmigen Bestandteile verdichtet und gereinigt. Zur Entfernung von H₂S und CO₂ wird in der Regel eine alkalische Wäsche, z. B. mit 5 bis 15 %iger Natronlauge, angewendet.

Vor der eigentlichen Aufarbeitung muss eine sorgfältige Trocknung durchgeführt werden, um die anschließende Tieftemperatur-Destillation nicht durch Eisbildung zu stören. Das trockene Rohgas wird dann in mehreren Stufen abgekühlt und in einem System von Kolonnen einer fraktionierten Destillation unterworfen.

Bei einem typischen Mitteldruck-Tieftemperaturverfahren wird das getrocknete Spaltgas auf -35 °C bis -45 °C abgekühlt und der sogenannten Methankolonne zugeführt, deren Kopftemperatur auf etwa -90 °C gehalten wird. Das Sumpfprodukt der Kolonne, das aus den C₂- und schwereren Kohlenwasserstoffen besteht, führt man einer weiteren Kolonne zu, in der die gesamte C₂-Fraktion als Kopfprodukt abgenommen wird, so dass das Sumpfprodukt im Wesentlichen aus C₃- und schwereren Kohlenwasserstoffen besteht. Das Kopfprodukt der Kolonne wird durch einen Kühler der sogenannten Ethylenkolonne zugeführt. Über Kopf wird reines Ethylen abgenommen, das Sumpfprodukt der Ethylensäule, im Wesentlichen Ethan, leitet man in der Regel wieder dem Spaltofen zu. Das nach Abtrennung der C₂-Fraktion erhaltene Sumpfprodukt, das aus C₃- und schwereren Kohlenwasserstoffen besteht, wird zur weiteren Trennung in eine weitere Kolonne eingeführt. Über Kopf geht eine C₃-Fraktion, die, gegebenenfalls nach selektiver Hydrierung der Propin- und Allen-Anteile, in der sogenannten C₃-Kolonne in Propylen bzw. Propan aufgetrennt wird. Propylen kann mit einer gewünschten Reinheit von bis zu 99,9 % isoliert werden.

Alternativ lassen sich Olefine durch Dehydrierung geeigneter Kohlenwasserstoff-Einsatzmaterialien im Wesentlichen ohne Spaltung von C-C-Bindungen herstellen. Hierbei kommt insbesondere die Dehydrierung von Propan zu Propylen und von Butan zu Butenen in Betracht. Die Dehydrierung erfolgt in der Regel unter Einsatz von Katalysatoren. Als Katalysator ist z. B. Chromoxid auf aktiver Tonerde als Träger, gegebenenfalls mit Zuschlägen, wie K₂O, CeO₂, SiO₂, TiO₂, ZrO₂, P₂O₅ usw., geeignet. Andererseits kommen auch Katalysatoren auf der Basis von calcium-Nickel-Phosphat in Betracht.

Die Dehydrierungseinheit besteht in der Regel aus einem oder mehreren mit Katalysator beschickten Reaktoren, z. B. Fließbettreaktoren. Das Gemisch aus frischem und gegebenenfalls rückgeführtem Kohlenwasserstoff-Einsatzmaterial wird vorgeheizt und strömt in den Reaktor ein. Das Reaktionsgas wird abgekühlt und komprimiert; aus den unkondensierbaren Anteilen können durch Wäsche mit geeigneten Lösungsmitteln Wertprodukte zurückgewonnen werden. Die verflüssigten Anteile können z. B. durch fraktionierte Destillation in einen Cᵢ-Olefin-angereicherten und einen Cᵢ-Olefin-abgereicherten Strom getrennt werden.

Der Cᵢ-Olefin-angereicherte Kohlenwasserstoffstrom wird zusammen mit Kohlenmonoxid und Wasserstoff in eine Hydroformylierungszone eingespeist.

Kohlenmonoxid und Wasserstoff werden üblicherweise in Form eines Gemisches, dem sogenannten Synthesegas, eingesetzt. Die Zusammensetzung des im erfindungsgemäßen Verfahren eingesetzten Synthesegases kann in weiten Bereichen variieren. Das molare Verhältnis von Kohlenmonoxid und Wasserstoff beträgt in der Regel 2:1 bis 1:2, insbesondere etwa 45:55 bis 50:50.

Die Temperatur bei der Hydroformylierungsreaktion liegt im Allgemeinen in einem Bereich von etwa 50 bis 200 °C, vorzugsweise etwa 60 bis 190 °C, insbesondere etwa 90 bis 190 °C. Die Umsetzung wird vorzugsweise bei einem Druck im Bereich von etwa 10 bis 700 bar, vorzugsweise 15 bis 200 bar, insbesondere 15 bis 60 bar durchgeführt. Der Reaktionsdruck kann in Abhängigkeit von der Aktivität des eingesetzten Hydroformylierungskatalysators variiert werden.

Geeignete druckfeste Reaktionsapparaturen für die Hydroformylierung sind dem Fachmann bekannt. Dazu zählen die allgemein üblichen Reaktoren für Gas-flüssig-Reaktionen, wie z. B. Gasumlaufreaktoren, Blasensäulen etc., die gegebenenfalls durch Einbauten unterteilt sein können.

Geeignete Hydroformylierungskatalysatoren sind die üblichen, dem Fachmann bekannten Übergangsmetallverbindungen und -komplexe, die sowohl mit als auch ohne Cokatalysatoren eingesetzt werden können. Bevorzugt handelt es sich bei dem Übergangsmetall um ein Metall der VIII. Nebengruppe des Periodensystems und insbesondere um Co, Ru, Rh, Pd, Pt, Os oder Ir, speziell um Rh, Co, Ir oder Ru.

Geeignete Komplexverbindungen sind beispielsweise die Carbonylverbindungen der genannten Metalle sowie Komplexe, deren Liganden ausgewählt sind unter Aminen, Arylphosphinen, Alkylphosphinen, Arylalkylphosphinen, Olefinen, Dienen etc. und Mischungen davon.

Es eignen sich z. B. Rhodiumkomplexe der allgemeinen Formel RhXₘL¹L² (L³)ₙ, worin
- X: für Halogenid, vorzugsweise Chlorid oder Bromid, Alkyl- oder Arylcarboxylat, Acetylacetonat, Aryl- oder Alkylsulfonat, insbesondere Phenylsulfonat und Toluolsulfonat, Hydrid oder das Diphenyltriazin-Anion,
- L¹, L², L³: unabhängig voneinander für CO, Olefine, Cycloolefine, vorzugsweise Cyclooctadien (COD), Dibenzophosphol, Benzonitril, PR₃ oder R₂P-A-PR₂, m für 1 oder 3 und n für 0, 1 oder 2 stehen. Unter R (die Reste R können gleich oder verschieden sein) sind Alkyl-, Cycloalkyl- und Arylreste zu verstehen, vorzugsweise Phenyl, p-Tolyl, m-Tolyl, p-Ethylphenyl, p-Cumyl, p-t-Butylphenyl, p-C₁-C₄-Alkoxyphenyl, vorzugsweise p-Anisyl, Xylyl, Mesityl, p-Hydroxyphenyl, das gegebenenfalls auch ethoxyliert vorliegen kann, Sulfophenyl, Isopropyl, C₁-C₄-Alkoxy, Cyclopentyl oder Cyclohexyl. A steht für 1,2-Ethylen oder 1,3-Propylen. Bevorzugt stehen L¹, L² oder L³ unabhängig voneinander für CO, COD, P(Phenyl)₃, P(i-Propyl)₃, P(Anisyl)₃, P(OC₂H₅)₃, P(Cyclohexyl)₃, Dibenzophosphol oder Benzonitril.
- X: steht bevorzugt für Hydrid, Chlorid, Bromid, Acetat, Tosylat, Acetylacetonat oder das Diphenyltriazin-Anion, insbesondere für Hydrid, Chlorid oder Acetat.

Bevorzugte Hydroformylierungskatalysatoren sind phosphorhaltige Rhodiumkatalysatoren, wie RhH(CO)₂(PPh₃)₂ oder RhH(CO)(PPh₃)₃.

Geeignete Hydroformylierungskatalysatoren werden z. B. in Beller et al., Journal of Molecular Catalysis A, 104 (1995), S. 17-85, beschrieben, worauf hier in vollem Umfang Bezug genommen wird.

In der Reaktionszone findet, bezogen auf das eingespeiste Olefin, pro Durchgang ein Teilumsatz statt. Der Umsatz beträgt im Allgemeinen 10 bis 90 % bezogen auf das eingespeiste Olefin.

Der Austrag aus der Reaktionszone wird einer ein- oder mehrstufigen Trennoperation unterzogen, wobei zumindest ein die Hauptmenge des Hydroformylierungsproduktes enthaltender Strom und ein im Wesentlichen aus nicht umgesetztem Olefin und gesättigtem Kohlenwasserstoff bestehender Strom erhalten werden. Je nach Austragsverfahren werden gegebenenfalls weitere Ströme erhalten, wie synthesegashaltige Abgase, hochsiedende Nebenprodukte der Hydroformylierung und/oder Hydroformylierungskatalysator enthaltende Ströme, die - gegebenenfalls nach Aufarbeitung - ganz oder teilweise in die Reaktionszone zurückgeführt oder aus dem Verfahren ausgeschleust werden. Vom Austrag aus der Reaktionszone können beispielsweise zuerst das Hydroformylierungsprodukt und gegebenenfalls höher als das Hydroformylierungsprodukt siedende Anteile abgetrennt werden. Anschließend kann ein Gemisch von nicht umgesetztem Olefin und gesättigtem Kohlenwasserstoff auskondensiert werden.

Zweckmäßigerweise erhält man den im Wesentlichen aus nicht umgesetztem Olefin und gesättigtem Kohlenwasserstoff bestehenden Strom jedoch dadurch, dass man vom Austrag aus der Reaktionszone zuerst ein rohes Hydroformylierungsprodukt abtrennt, welches nicht umgesetztes Olefin und gesättigten Kohlenwasserstoff gelöst enthält, und das rohe Hydroformylierungsprodukt dann einem Entgasungsschritt unterzieht, bei dem ein Strom anfällt, der im Wesentlichen aus nicht umgesetztem Olefin und gesättigtem Kohlenwasserstoff besteht. Der vom rohen Hydroformylierungsprodukt befreite Reaktionsaustrag wird in der Regel ganz oder teilweise in die Reaktionszone zurückgeführt. Zur Entgasung kann das rohe Hydroformylierungsprodukt entspannt, erwärmt und/oder mit einem Strippgas, wie Synthesegas oder Stickstoff, behandelt werden. Zweckmäßigerweise führt man die Entgasung in einer Kolonne durch, wobei das rohe Hydroformylierungsprodukt im Bereich der Kolonnenmitte eingespeist wird, am Kolonnensumpf das entgaste Hydroformylierungsprodukt abgezogen wird, das der weiteren Aufarbeitung zugeführt wird, und am Kolonnenkopf ein flüssiger oder gasförmiger Strom abgezogen wird, der im Wesentlichen aus nicht umgesetztem Olefin und gesättigtem Kohlenwasserstoff besteht.

Die Abtrennung des rohen Hydroformylierungsproduktes vom Austrag aus der Reaktionszone kann auf verschiedene Weise erfolgen. Es kann zum einen das sogenannte Flüssigaustragsverfahren zum Einsatz kommen, bei dem der im Wesentlichen, bis auf das im Überschuss zur Hydroformylierung eingesetzte Synthesegas, flüssige Austrag aus der Reaktionszone entspannt wird, wobei in Folge der Druckerniedrigung der Austrag in eine Flüssigphase, die im Wesentlichen aus hochsiedenden Nebenprodukten, dem homogen gelösten Hydroformylierungskatalysator und geringen Mengen an Hydroformylierungsprodukt, an nicht umgesetztem Olefin und an gesättigtem Kohlenwasserstoff besteht, und eine Gasphase, die im Wesentlichen aus Hydroformylierungsprodukt, nicht umgesetztem Olefin und gesättigtem Kohlenwasserstoff sowie nicht umgesetztem Synthesegas besteht, aufgetrennt wird. Die Flüssigphase kann als Rückführstrom wieder in den Reaktor geleitet werden. Durch zumindest partielle Kondensation der Gasphase wird das rohe Hydroformylierungsprodukt erhalten. Die bei der Kondensation zurückbleibende Gasphase wird ganz oder teilweise in die Reaktionszone zurückgeführt.

Mit Vorteil kann die in der Entspannungsstufe primär anfallende Gas- und Flüssigphase nach dem in der WO 97/07086 beschriebenen Verfahren aufgearbeitet werden. Zu diesem Zweck wird die Flüssigphase erwärmt und in den oberen Bereich einer Kolonne eingeleitet, während die Gasphase in den Sumpf der Kolonne eingeleitet wird. Flüssigphase und Gasphase werden dadurch im Gegenstrom geführt. Um den gegenseitigen Kontakt der Phasen zu erhöhen, ist die Kolonne vorzugsweise mit Füllkörpern gefüllt. Durch den innigen Kontakt der Gasphase mit der Flüssigphase werden die in der Flüssigphase vorhandenen Restmengen an Hydroformylierungsprodukt, nicht umgesetztem Olefin und gesättigtem Kohlenwasserstoff in die Gasphase transferiert, so dass der die Kolonne am Kopf verlassende Gasstrom im Vergleich zu dem am unteren Ende der Kolonne eingeführten Gasstrom an Hydroformylierungsprodukt, nicht umgesetztem Olefin und gesättigtem Kohlenwasserstoff angereichert ist. Die weitere Aufarbeitung des die Kolonne verlassenden Gasstroms und der die Kolonne verlassenden Flüssigphase erfolgt in üblicher Weise, beispielsweise wie oben beschrieben.

Alternativ kann man nach dem sogenannten Kreisgasverfahren (gas recycle) verfahren, bei dem aus dem Gasraum des Hydroformylierungsreaktors ein Gasstrom abgezogen wird. Dieser Gasstrom besteht im Wesentlichen aus Synthesegas, nicht umgesetztem Olefin und gesättigtem Kohlenwasserstoff, wobei nach Maßgabe des Dampfdrucks im Hydroformylierungsreaktor das bei der Hydroformylierungsreaktion gebildete Hydroformylierungsprodukt mitgeführt wird. Aus dem Gasstrom wird das mitgeführte Hydroformylierungsprodukt z. B. durch Abkühlen auskondensiert, und der vom Flüssiganteil befreite Gasstrom wird zurück in den Hydroformylierungsreaktor geführt.

Der im Wesentlichen aus nicht umgesetztem Olefin und gesättigtem Kohlenwasserstoff bestehende Strom enthält z. B. 50 bis 95 Gew.-%, vorzugsweise 60 bis 80 Gew.-%, Olefin und 5 bis 50 Gew.-%, vorzugsweise 20 bis 40 Gew.-% gesättigten Kohlenwasserstoff.

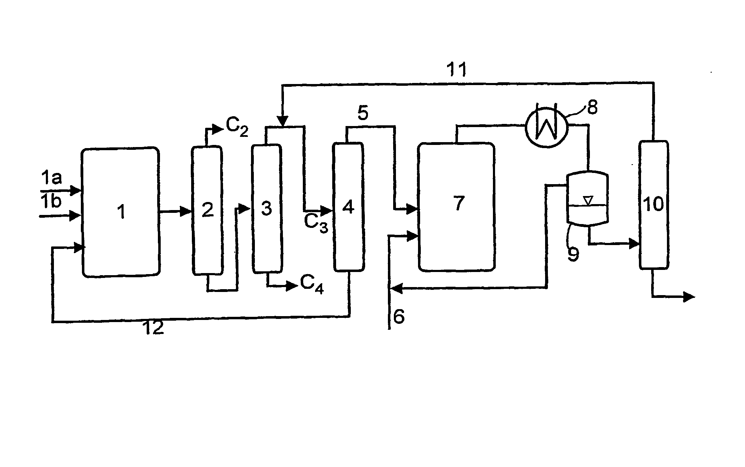

Die beigefügte Fig. 1 zeigt schematisch eine zur Durchführung des erfindungsgemäßen Verfahrens geeignete Anlage zur Herstellung von Hydroformylierungsprodukten des Propylens. An sich selbstverständliche Anlagendetails, die für das Verständnis der vorliegenden Erfindung nicht erforderlich sind, wurden weggelassen. Über die Leitungen (la) und (1b) werden vorverdampftes Naphtha und überhitzter Wasserdampf in einen Röhren-Spaltofen (1) eingebracht. Die Spaltprodukte werden nach dem Quenchen und Trocknen einer Trennkolonne (2) zugeführt, in der die gesamte C₂-Fraktion als Kopfprodukt abgenommen wird. Das Sumpfprodukt der Kolonne (2), das im Wesentlichen aus C₃- und schweren Kohlenwasserstoffen besteht, wird zur weiteren Trennung in eine Kolonne (3) eingeführt. Über Kopf geht eine C₃-Fraktion, die in der C₃-Kolonne (4) in einen Propylenstrom (5) am Kopf und einen Propanstrom am Sumpf aufgetrennt wird. Der Propanstrom wird über die Leitung (12) wieder dem Spaltofen zugeführt. Der Propylenstrom (5) wird einem Hydroformylierungsreaktor (7) zugeleitet, dem über die Leitung (6) außerdem Synthesegas zugeführt wird. Das Propylen wird im Hydroformylierungsreaktor zu Butyraldehyd umgesetzt. Aus dem Gasraum des Hydroformylierungsreaktors (7) wird ein Gasstrom abgezogen, der im Kühler (8) gekühlt und einem Phasentrenngefäß (9) zugeführt wird. Die gasförmigen Anteile aus dem Phasentrenngefäß (9), die im Wesentlichen aus nicht umgesetzten Synthesegas, nicht umgesetzten Propylen und Propan bestehen, werden wieder dem Hydroformylierungsreaktor zugeführt. Der Flüssiganteil aus dem Phasentrenngefäß (9), der im Wesentlichen aus Butyraldehyd mit darin gelöstem Propylen und Propan besteht, wird einer Entgasungskolonne (10) zugeleitet, an deren Sumpf weitgehend gasfreier Butyraldehyd anfällt. Das am Kopf der Entgasungskolonne (10) anfallende Gemisch aus Propylen und Propan wird mit dem Kopfabzug der Kolonne (3) vereinigt und gemeinsam der C₃-Kolonne (4) des Steamcrackers zugeführt.

Die Erfindung wird durch das folgende Beispiel näher veranschaulicht.

### Beispiel

Es wurde eine Anlage verwendet, wie sie in Fig. 1 dargestellt ist. Dem Hydroformylierungsreaktor (7) wurden ein Zulauf von 10 t/h "chemical grade propylen" (95 Gew.-% Propylen und 5 Gew.-% Propan), das am Kopf der C₃-Kolonne (4) eines Steamcrackers anfiel, sowie das für die Umsetzung notwendige Synthesegas zugeführt. Das gebildete Produkt, ein Gemisch aus n- und iso-Butyraldehyd, wurde zusammen mit dem nicht umgesetzten Propylen sowie dem zugeführten und dem bei der Reaktion gebildeten Propan mit Hilfe eines Kreisgasstroms aus dem Reaktor ausgetragen. Die kondensierbaren Anteile wurden in dem Kühler (8) kondensiert und im Abscheider (9) gesammelt. Die Flüssigphase enthielt 78,3 Gew.-% Butyraldehyd, 14,3 Gew.-% Propylen und 7,4 Gew.-% Propan. Sie wurde der Entgasungskolonne (10) zugeführt (20,3 t/h), wo sie in einen C₃-freien Aldehydstrom am Sumpf (15,9 t/h) und ein Gemisch aus 66 Gew.-% Propylen und 34 Gew.-% Propan am Kopf (4,4 t/h) aufgetrennt wurde.

Dieses Gemisch wurde mit dem Zulauf zur C₃-Trennkolonne (4) des Steamcrackers vereinigt, der am Kopf der davorliegenden C₃/C₄-Trennkolonne (3) abgenommen wurde. In der C₃-Trennkolonne (4) wurde das Gemisch in einen praktisch propylenfreien Propanstrom am Sumpf und ein Gemisch aus 95 Gew.-% Propylen und 5 Gew.-% Propan am Kopf aufgetrennt. Der aus der Entgasungskolonne mit (10) stammende Propylen/Propan-Strom hatte einen Anteil von 3,1 t/h am Kopfprodukt und von 1,3 t/h am Sumpfabzug der C₃-Trennkolonne.

Der praktisch propylenfreie Propanstrom vom Sumpf der C₃-Trennkolonne (4) wurde dem Spaltofen des Crackers wieder als Einsatzstoff zugeführt.

## Patentansprüche

1. Verfahren zur Herstellung von Hydroformylierungsprodukten von Olefinen mit 2 bis 8 Kohlenstoffatomen, bei dem man
a) ein Kohlenwasserstoff-Einsatzmaterial in eine Crack/Dehydrierzone einspeist und einer thermischen und/oder katalytischen Crackung und/oder Dehydrierung unter Erhalt eines olefinhaltigen Spaltgases unterwirft,
b) das Spaltgas oder Fraktionen davon einer Auftrennung in wenigstens einen Cᵢ-Olefin-angereicherten Kohlenwasserstoffstrom und wenigstens einen Cᵢ-Olefin-abgereicherten Kohlenwasserstoffstrom unterzieht,
c) den Cᵢ-Olefin-angereicherten Kohlenwasserstoffstrom mit Kohlenmonoxid und Wasserstoff in eine Hydroformylierungszone einspeist und in Gegenwart eines Hydroformylierungskatalysators umsetzt,
d) aus dem Austrag aus der Hydroformylierungszone einen im Wesentlichen aus nicht umgesetztem Cᵢ-Olefin und gesättigtem Cᵢ-Kohlenwasserstoff bestehenden Strom abtrennt,
e) den im Wesentlichen aus nicht umgesetztem Cᵢ-Olefin und gesättigtem Cᵢ-Kohlenwasserstoff bestehenden Strom zumindest teilweise in den Schritt b) zurückführt, wobei i für eine ganze Zahl von 2 bis 8 steht.

2. Verfahren nach Anspruch 1, bei dem man den Cᵢ-Olefin-abgereicherten Kohlenwasserstoffstrom zumindest teilweise in die Crack/Dehydrierzone zurückführt.

3. Verfahren nach Anspruch 1 oder 2, bei dem die Auftrennung in Schritt b) wenigstens einen Rektifikationsschritt umfasst.

4. Verfahren nach einem der vorhergehenden Ansprüche, bei dem es sich bei dem Cᵢ-Olefin um Propylen handelt.

5. Verfahren nach einem der vorhergehenden Ansprüche, bei dem man den im Wesentlichen aus nicht umgesetztem Cᵢ-Olefin und gesättigtem Cᵢ-Kohlenwasserstoff bestehenden Strom **dadurch** erhält, dass man vom Austrag aus der Hydroformylierungszone zuerst ein rohes Hydroformylierungsprodukt isoliert, das nicht umgesetztes Cᵢ-Olefin und gesättigten Cᵢ-Kohlenwasserstoff gelöst enthält, und dieses einem Entgasungsschritt unterwirft.

6. Verfahren nach einem der vorhergehenden Ansprüche, bei dem es sich bei dem Kohlenwasserstoff-Einsatzmaterial um Naphtha oder Erdgas oder Fraktionen davon handelt.

7. Verfahren nach einem der vorhergehenden Ansprüche, bei dem man die thermische Crackung in Gegenwart von Wasserdampf durchführt.

## Claims

1. A process for preparing hydroformylation products of olefins having from 2 to 8 carbon atoms, which comprises
a) feeding a hydrocarbon feedstock into a cracking/dehydrogenation zone and subjecting it to thermal and/or catalytic cracking and/or dehydrogenation to give an olefin-containing cracker gas,
b) subjecting the cracker gas or fractions thereof to a fractionation to give at least one Cᵢ-olefin-enriched hydrocarbon stream and at least one Ci-olefin-depleted hydrocarbon stream,
c) feeding the Cᵢ-olefin-enriched hydrocarbon stream together with carbon monoxide and hydrogen into a hydroformylation zone and reacting it in the presence of a hydroformylation catalyst,
d) separating a stream consisting essentially of unreacted Cᵢ-olefin and saturated Cᵢ-hydrocarbon from the output from the hydroformylation zone,
e) recirculating at least part of the stream consisting essentially of unreacted Ci-olefin and saturated Cᵢ-hydrocarbon to step b), where i is an integer from 2 to 8.

2. The process according to claim 1, wherein at least part of the Cᵢ-olefin-depleted hydrocarbon stream is recirculated to the cracking/dehydrogenation zone.

3. A process according to claim 1 or 2, wherein the fractionation in step b) comprises at least one rectification step.

4. A process according to any of the preceding claims, wherein the Ci-olefin is propylene.

5. A process according to any of the preceding claims, wherein the stream consisting essentially of unreacted Ci-olefin and saturated Cᵢ-hydrocarbon is obtained by firstly isolating a crude hydroformylation product in which unreacted Ci-olefin and saturated Cᵢ-hydrocarbon are comprised in dissolved form from the output from the hydroformylation zone and subjecting this crude hydroformylation product to a degassing step.

6. A process according to any of the preceding claims, wherein the hydrocarbon feedstock is naphtha or natural gas or fractions thereof.

7. A process according to any of the preceding claims, wherein thermal cracking is carried out in the presence of water vapor.

## Revendications

1. Procédé de préparation de produits d'hydroformylation d'oléfines comportant 2 à 8 atomes de carbone, dans lequel
a) on alimente en une matière de chargement à base d'hydrocarbure une zone de craquage/déshydrogénation et on la soumet à un craquage thermique et/ou catalytique et/ou à une déshydrogénation, avec obtention d'un gaz effluent contenant de l'oléfine,
b) on soumet le gaz effluent ou des fractions de celui-ci à une séparation en au moins un courant d'hydrocarbure enrichi en oléfine en Ci et en au moins un courant d'hydrocarbure appauvri en oléfine en Cᵢ,
c) on injecte le courant d'hydrocarbure enrichi en oléfine en Ci avec du monoxyde de carbone et de l'hydrogène dans une zone d'hydroformylation et on les fait réagir en présence d'un catalyseur d'hydroformylation,
d) on isole, à partir de l'effluent de la zone d'hydroformylation, un courant constitué essentiellement d'oléfine en Ci n'ayant pas réagi et d'hydrocarbure en Ci saturé,
e) on recycle le courant constitué essentiellement d'oléfine en Cᵢ n'ayant pas réagi et d'hydrocarbure en Cᵢ saturé au moins partiellement dans l'étape b), i représentant un nombre entier de 2 à 8.

2. Procédé suivant la revendication 1, dans lequel on recycle au moins partiellement le courant d'hydrocarbure appauvri en oléfine en Cᵢ dans la zone de craquage/déshydrogénation.

3. Procédé suivant la revendication 1 ou 2, dans lequel la séparation de l'étape b) comprend au moins une étape de rectification.

4. Procédé suivant l'une des revendications précédentes, dans lequel, en ce qui concerne l'oléfine en Ci, il s'agit de propylène.

5. Procédé suivant l'une des revendications précédentes, dans lequel on obtient le courant constitué essentiellement d'oléfine en Ci n'ayant pas réagi et d'hydrocarbure en Ci saturé par le fait qu'on isole de l'effluent de la zone d'hydroformylation tout d'abord un produit d'hydroformylation brut, qui contient sous forme dissoute de l'oléfine en Ci n'ayant pas réagi et de l'hydrocarbure en Cᵢ saturé, et on soumet celui-ci à une étape de dégazage.

6. Procédé suivant l'une des revendications précédentes, dans lequel, en ce qui concerne la matière de chargement à base d'hydrocarbure, il s'agit de naphte ou de gaz naturel ou de leurs fractions.

7. Procédé suivant l'une des revendications précédentes, dans lequel on effectue le craquage thermique en présence de vapeur d'eau.
